# EUROPÄISCHE PATENTSCHRIFT

(11) **EP 2 361 079 B1**
(45) Veröffentlichungstag und Bekanntmachung des Hinweises auf die Patenterteilung: **31.05.2017**
(21) Anmeldenummer: 09760877.2
(22) Anmeldetag: 30.11.2009
(51) Int. Cl.: A61K 8/22, A61K 8/46, A61Q 5/04, A61Q 5/10, A61K 8/44, A61K 8/23

(54) **VERFAHREN ZUM FÄRBEN UND VERFORMEN KERATINHALTIGER FASERN**
METHOD FOR DYEING AND SHAPING KERATIN FIBERS
PROCÉDÉ DE COLORATION ET DE MISE EN FORME DE FIBRES KÉRATINIQUES

(30) Priorität: 22.12.2008 DE 102008064218
(43) Veröffentlichungstag der Anmeldung: 31.08.2011
(73) Patentinhaber: Henkel AG & Co. KGaA, 40589 Düsseldorf (DE)
(72) Erfinder: EMMERLING, Winfried, 25436 Tornesch (DE); RAUTENBERG-GROTH, Birgit, 25479 Ellerau (DE)
(86) Internationale Anmeldenummer: PCT/EP2009/066046
(87) Internationale Veröffentlichungsnummer: WO 2010/072514

(56) Entgegenhaltungen:
- EP-A1- 1 426 040
- EP-A1- 1 655 056
- EP-A1- 2 020 255
- EP-A1- 2 191 865
- EP-A2- 0 079 540
- US-B1- 6 391 063
- Attenberger et al.: "Fachkunde für Frisöre" 1992, Kieser Verlag GmbH , XP002586160 ISBN: 382422125X , Seiten 163-196 Seite 196, Spalte 1, Zeile 13 - Zeile 20 Seite 196, Spalte 3, Zeile 19 - Zeile 34

## Beschreibung

Die Erfindung betrifft ein Verfahren zum gleichzeitigen Färben und dauerhaften Verformen keratinhaltiger Fasern, insbesondere menschlicher Haare.

Als keratinhaltige Fasern können prinzipiell alle tierischen Haare, z.B. Wolle, Rosshaar, Angorahaar, Pelze, Federn und daraus gefertigte Produkte oder Textilien eingesetzt werden. Bevorzugt wird die Erfindung jedoch im Rahmen einer Haarumformung, insbesondere der Dauerwellung glatter oder der Glättung krauser menschlicher Haare und daraus gefertigter Perücken, eingesetzt.

Eine dauerhafte Verformung keratinhaltiger Fasern wird üblicherweise derart durchgeführt, dass man die Faser mechanisch verformt und die Verformung durch geeignete Hilfsmittel festlegt. Vor und/oder nach dieser Verformung behandelt man die Faser mit einer keratinreduzierenden Zubereitung. Nach einem Spülvorgang wird die Faser dann in dem so genannten Fixierschritt mit einer Oxidationsmittelzubereitung behandelt, gespült und nach oder während des Fixierschritts von den Verformungshilfsmitteln (Wicklern, Papilloten) befreit. Wenn als keratinreduzierende Komponente ein Merkaptan, z.B. Ammoniumthioglykolat, verwendet wird, spaltet dieses einen Teil der Disulfid-Brücken des Keratin-Moleküls zu -SH-Gruppen, so dass es zu einer Erweichung der Keratinfaser kommt. Bei der späteren oxidativen Fixierung werden erneut Disulfid-Brücken im Haarkeratin geknüpft, so dass das Keratingefüge in der vorgegebenen Verformung fixiert wird. Alternativ ist es bekannt, zur Haarverformung anstelle der Merkaptane Sulfit zu verwenden. Durch Hydrogensulfit-Lösungen und/oder Sulfit-Lösungen und/oder Disulfit-Lösungen werden Disulfid-Brücken des Keratins in einer Sulfitolyse nach der Gleichung

R - S - S - R + HSO₃⁽⁻⁾ → R - SH + R - S - SO₃⁽⁻⁾

gespalten und auf diese Weise eine Erweichung der Keratinfaser erreicht. Hydrogensulfit-, sulfit- bzw. disulfithaltige Reduktionsmittel weisen nicht den starken Eigengeruch der merkaptanhaltigen Mittel auf. Die Spaltung kann wie zuvor geschildert in einem Fixierschritt mit Hilfe eines Oxidationsmittels unter Bildung von neuen Disulfid-Brücken wieder rückgängig gemacht werden.

Die permanente Glättung keratinhaltiger Fasern wird analog durch den Einsatz von keratinreduzierenden und -oxidierenden Zusammensetzungen erzielt. In einem entsprechenden Verfahren wird das krause Haar entweder auf Wickler mit einem großen Durchmesser von üblicherweise mehr als 15 mm gewickelt oder das Haar unter Einwirkung der keratinreduzierenden Zusammensetzung glatt gekämmt. Anstelle des Wicklers ist es auch möglich, die Faser auf ein Glättungsboard glatt zu legen. Glättungsboarde sind üblicherweise rechteckige Tafeln z.B. aus Kunststoff.

Wenn zusätzlich zur Umformung auch eine Färbung der keratinischen Faser gewünscht wird, kann die Färbung als separate Behandlung vor oder nach der erfolgten Umformung durchgeführt werden. Insbesondere im Falle einer oxidativen Färbung führt dies aber zu einer extremen Beanspruchung der Faser, da jede oxidative Behandlung der Faser deren innere Struktur schädigt. Zudem ist ein solches Vorgehen sehr zeitaufwändig.

Es wurden daher bereits einige Verfahren zur gleichzeitigen Umformung und Färbung keratinischer Fasern, insbesondere Haare, vorgeschlagen. In vielen Fällen wird dazu im Fixierschritt eine Oxidationsmittelzubereitung eingesetzt, die neben dem Oxidationsmittel einen direktziehenden Farbstoff und/oder ein Oxidationsfarbstoffvorprodukt enthält. Ein entsprechendes Vorgehen ist beispielsweise in DE 197 13 698 C1 beschrieben. Dieses Vorgehen hat jedoch den Nachteil, dass die Färbung gleichzeitig mit der Fixierung, d.h. zu einem Zeitpunkt erfolgt, zu dem die zu behandelnden Fasern auf ein Verformungshilfsmittel aufgebracht sind und dadurch unter mechanischer Spannung stehen. Dies behindert das gleichmäßige Aufbringen der Farbstoffe, so dass die Gefahr eines ungleichmäßigen Färbeergebnisses besteht.

Aus EP 0 352 375 A1 und EP 1 287 812 A2 sind Verfahren zur gleichzeitigen Umformung und Färbung von Haaren bekannt, bei denen eine keratinreduzierende Zubereitung eingesetzt wird, die bereits die notwendigen Farbstoffe und/oder Farbstoffvorprodukte enthält. Zumindest ein Teil der jeweiligen keratinreduzierenden Zubereitung wird auf das Haar aufgebracht, nachdem dieses mechanisch verformt wurde.

Aus EP 1 655 056 A1 ist ein Verfahren zur Umformung von Haaren bekannt, in welchem das Haar zuerst mit einem oxidativen Färbemittel gefärbt und danach gespült wird, sodann eine Zwischenbehandlung bei einem pH-Wert von 2 bis 6 vorgenommen und danach gespült wird und daran ein Dauerwellverfahren anschließt.

EP 0 079 540 A2 und US 6 391 063 B1 betreffen Haarfärbemittel, welche jeweils spezielle Oxidationsfarbstoffvorprodukte enthalten, deren Färbungen durch die anschließende Einwirkung von Dauerwellpräparaten keine Farbveränderung erleiden sollen. Der Einsatz von kationischen Polymeren im Färbemittel wird in keinem der beiden Dokumente offenbart.

Aufgabe der Erfindung ist es, ein Umformungs- und Färbeverfahren für keratinhaltige Fasern, insbesondere für menschliches Haar, bereitzustellen, das wenig Verfahrensschritte aufweist, ein zum Stand der Technik vergleichbares oder besseres Umformungsergebnis liefert und die Faser gleichmäßig im gewünschten Farbton gefärbt wird.

Überraschenderweise wurde gefunden, dass die Aufgabe durch ein Verfahren gelöst wird, bei dem die keratinhaltigen Fasern, insbesondere menschlichen Haare, zunächst mit einem Oxidationsfärbemittel behandelt, dann gespült und danach mit Unterstützung einer Reduktionsmittelzubereitung, unter Zuhilfenahme von Verformungshilfsmitteln verformt werden.

Ein erster Gegenstand der Erfindung ist daher ein Verfahren zum gleichzeitigen Färben und dauerhaften Verformen keratinhaltiger Fasern, insbesondere menschlicher Haare, wobei
die keratinhaltigen Fasern vor dem Schritt (i) angefeuchtet werden,
(i) eine wässrige Zusammensetzung, enthaltend mindestens ein Oxidationsfarbstoffvorprodukt, mindestens ein kationisches Polymer und Wasserstoffperoxid, auf die Fasern aufgetragen wird,
(ii) die Fasern nach einer Einwirkzeit Z1 gespült und gegebenenfalls getrocknet werden,
(iii) sodann die Fasern unter Zuhilfenahme von Verformungshilfsmitteln verformt werden,
(iv) sowie eine wässrige Zusammensetzung, enthaltend mindestens eine keratinreduzierende Verbindung (nachfolgend Wellmittel genannt) auf die Fasern aufgetragen wird,
(v) die Fasern nach einer Einwirkzeit Z2 gespült und gegebenenfalls getrocknet werden,
(vi) schließlich eine oxidierende Zusammensetzung, enthaltend mindestens eine oxidierende Verbindung (nachfolgend Fixiermittel genannt), auf die Fasern aufgetragen und nach einer Einwirkzeit Z3 wieder abgespült wird,
mit der Maßgabe, dass
- Schritt (iii) unmittelbar auf Schritt (ii) folgt,
- Schritt (iv) unmittelbar auf Schritt (iii) folgt oder die Schritte (iii) und (iv) gleichzeitig ablaufen.

Das erfindungsgemäße Verfahren zeichnet sich insbesondere durch eine besondere Gleichmäßigkeit der Umformung, sowie ein gleichmäßiges und intensives Färbeergebnis des Wunschfarbtons aus.

Verformungshilfsmittel im Sinne des erfindungsgemäßen Verfahrens können
- z.B. Lockenwickler oder Papilloten im Falle einer Dauerwelle,
- oder Hilfsmittel für eine mechanische Glättung, wie ein Kamm oder eine Bürste, ein Glättungsboard oder ein beheizbares Glättungseisen im Falle einer Haarglättung sein.

Wenn die Verformungshilfsmittel, beispielsweise Wickler, im Rahmen eines Dauerwellverfahrens für einen längeren Zeitraum an der Faser befestigt werden, so kann es zweckmäßig sein, diese Verformungshilfsmittel vor, während oder nach Schritt (vi) zu entfernen. Es kann in diesem Zusammenhang vorteilhaft sein, die Verformungshilfsmittel während des Schritts (vi) im Haar zu belassen, sie danach zu entfernen und danach Schritt (vi) als sogenannten Nachfixierschritt (vii) zu wiederholen.

Ein entscheidender Schritt des erfindungsgemäßen Verfahrens ist, dass die oxidative Färbung unmittelbar vor der Anwendung des Wellmittels liegt und keine Zwischenbehandlung zwischen Färbung und Applikation des Wellmittels erfolgt. Unmittelbar bedeutet im Sinne der Erfindung, dass keine weiteren Verfahrensschritte dazwischen liegen und dass zwischen dem Ende des einen und dem Beginn des nächsten Schrittes nicht mehr als 30 Minuten, insbesondere nicht mehr als 15 Minuten, liegen.

Die keratinhaltigen Fasern werden vor dem Schritt (i) angefeuchtet. Dies kann durch Besprühen der Fasern mit einer Flüssigkeit, bevorzugt mit Wasser, geschehen. Bevorzugterweise werden die Fasern vor Schritt (i) mit einem herkömmlichen Shampoo shampooniert, gespült und dann mit einem Handtuch frottiert. Nach Abschluß des Frottierschritts bleibt eine fühlbare Restfeuchtigkeit im Haar zurück.

Die Einwirkzeit Z1 beträgt bevorzugt 5 bis 60 Minuten, besonders bevorzugt 10 bis 30 Minuten. Die Einwirkzeit Z2 beträgt bevorzugt 5 bis 60 Minuten, besonders bevorzugt 10 bis 30 Minuten. Die Einwirkzeit Z3 beträgt bevorzugt 1 bis 30 Minuten, besonders bevorzugt 5 bis 20 Minuten.

Eine wässrige Zusammensetzung im Sinne der Erfindung enthält mindestens 50 Gew.% Wasser bezogen auf das Gewicht der gesamten Zusammensetzung. Diese wässrige Zusammensetzung kann in verschiedenen Formen, beispielsweise als Lotion, Öl-in-Wasser-Emulsion oder Wasser-in-Öl-Emulsion, vorliegen.

Die wässrige Zusammensetzung des Schritts (i) enhält neben Wasserstoffperoxid zwingend mindestens ein kationisches Polymer und mindestens ein Oxidationsfarbstoffvorprodukt. Oxidationsfarbstoffvorprodukte unterteilen sich in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 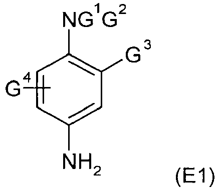 wobei
- G¹ steht für ein Wasserstoffatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen 4'-Aminophenylrest oder einen (C₁ bis C₄)-Alkylrest, der mit einer stickstoffhaltigen Gruppe, einem Phenyl- oder einem 4'-Aminophenylrest substituiert ist;
- G² steht für ein Wasserstoffatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest oder einen (C₁ bis C₄)-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Hydroxyalkoxyrest, einen (C₁ bis C₄)-Alkoxy-(C₁ bis C₄)-alkylrest, einen (C₁ bis C₄)-Acetylaminoalkoxyrest, einen Mesylamino-(C₁ bis C₄)-alkoxyrest oder einen (C₁ bis C₄)-Carbamoylaminoalkoxyrest;
- G⁴ steht für ein Wasserstoffatom, ein Halogenatom, einen (C₁ bis C₄)-Alkylrest oder einen (C₁ bis C₄)-Alkoxy-(C₁ bis C₄)-alkylrest, oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Besonders bevorzugte p-Phenylendiamine der Formel (E1) werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(a,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-Methoxymethyl-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1 H-imidazol-1-yl)propyl]amin, 5,8-Diaminobenzo-1,4-dioxan sowie ihren physiologisch verträglichen Salzen.

Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-(β-Hydroxyethyl)-p-phenylendiamin, 2-(a,β-Dihydroxyethyl)-p-phenylendiamin, N,N-Bis-(β-hydroxyethyl)-p-phenylendiamin, 2-Methoxymethyl-p-phenylendiamin, N-(4-Amino-3-methylphenyl)-N-[3-(1 H-imidazol-1-yl)propyl]amin, sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 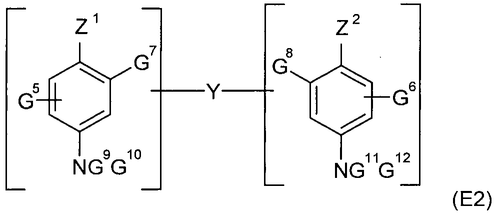 wobei:
- Z¹ und Z² stehen unabhängig voneinander für einen Hydroxyl- oder NH₂-Rest, der gegebenenfalls durch einen (C₁ bis C₄)-Alkylrest, durch einen (C₁ bis C₄)-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder (C₁ bis C₈)-Alkoxyreste substituiert sein kann, oder eine direkte Bindung,
- G⁵ und G⁶ stehen unabhängig voneinander für ein Wasserstoff- oder Halogenatom, einen _{(C1 bis C6)-} bis C₄)-Alkylrest, einen (C₁ bis C₄)-Monohydroxyalkylrest, einen (C₂ bis C₄)-Polyhydroxyalkylrest, einen (C₁ bis C₄)-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen (C₁ bis C₄)-Alkylrest,
mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) werden insbesondere aus mindestens einer der folgenden Verbindungen ausgewählt: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4'-aminophenyl)-tetramethylendiamin, N,N'-Bis-(β-hydroxyethyl)-N,N'-bis-(4'-aminophenyl)-tetramethylendiamin, N,N'-Bis-(4-(methylamino)phenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze.

Ganz besonders bevorzugte zweikernige Entwicklerkomponenten der Formel (E2) werden ausgewählt unter N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 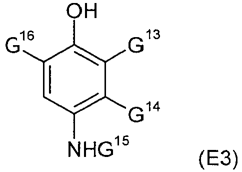 wobei:
- G¹³ steht für ein Wasserstoffatom, ein Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen (C₁ bis C₄)-Aminoalkylrest, einen Hydroxy-(C₁ bis C₄)-alkylaminorest, einen (C₁ bis C₄)-Hydroxyalkoxyrest, einen (C₁ bis C₄)-Hydroxyalkyl-(C₁ bis C₄)-aminoalkylrest oder einen (Di-[(C₁ bis C₄)-alkyl]amino)-(C₁ bis C₄)-alkylrest, und
- G¹⁴ steht für ein Wasserstoff- oder Halogenatom, einen (C₁ bis C₄)-Alkylrest, einen (C₁ bis C₄)-Monohydroxyalkylrest, einen (C₁ bis C₄)-Polyhydroxyalkylrest, einen (C₁ bis C₄)-Alkoxy-(C₁ bis C₄)-alkylrest, einen (C₁ bis C₄)-Aminoalkylrest oder einen (C₁ bis C₄)-Cyanoalkylrest,
- G¹⁵ steht für Wasserstoff, einen (C₁ bis C₄)-Alkylrest, einen (C₁ bis C₄)-Monohydroxyalkylrest, einen (C₂ bis C₄)-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen.

Bevorzugte Pyrimidin-Derivate werden erfindungsgemäß ausgewählt aus Verbindungen gemäß Formel (E4) bzw. deren physiologisch verträglichen Salzen, 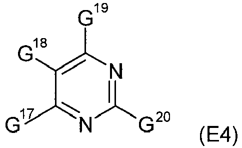 worin
- G¹⁷, G¹⁸ und G¹⁹ unabhängig voneinander für ein Wasserstoffatom, eine Hydroxygruppe, eine (C₁ bis C₄)-Alkoxygruppe oder eine Aminogruppe steht und
- G²⁰ für eine Hydroxygruppe oder eine Gruppe -NG²¹G²² steht, worin G²¹ und G²² unabhängig voneinander stehen für ein Wasserstoffatom, eine (C₁ bis C₄)-Alkylgruppe, eine (C₁ bis C₄)-Monohydroxyalkylgruppe,
mit der Maßgabe, dass maximal zwei der Gruppen aus G¹⁷, G¹⁸, G¹⁹ und G²⁰ eine Hydroxygruppe bedeuten und höchstens zwei der Reste G¹⁷, G¹⁸ und G¹⁹ für ein Wasserstoffatom stehen. Dabei ist es wiederum bevorzugt, wenn gemäß Formel (E4) mindestens zwei Gruppen aus G¹⁷, G¹⁸, G¹⁹ und G²⁰ für eine Gruppe -NG²¹G²² stehen und höchstens zwei Gruppen aus G¹⁷, G¹⁸, G¹⁹ und G²⁰ für eine Hydroxygruppe stehen.

Besonders bevorzugte Pyrimidin-Derivate sind insbesondere die Verbindungen 2,4,5,6-Tetraamino-pyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate werden erfindungsgemäß ausgewählt aus Verbindungen gemäß Formel (E5), 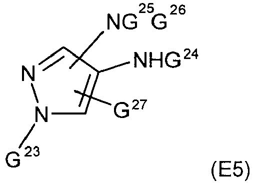 worin - G²³, G²⁴, G²⁵ stehen unabhängig voneinander für ein Wasserstoffatom, eine (C₁ bis C₄)-Alkylgruppe, eine (C₁ bis C₄)-Monohydroxyalkylgruppe, eine (C₂ bis C₄)-Polyhydroxyalkylgruppe, eine gegebenenfalls substituierte Arylgruppe oder eine gegebenenfalls substituierte Aryl-(C₁ bis C₄)-alkylgruppe, mit der Maßgabe dass, wenn G²⁵ für ein Wasserstoffatom steht, G²⁷ neben den vorgenannten Gruppen zusätzlich für eine Gruppe -NH₂ stehen kann,
- G²⁶ steht für ein Wasserstoffatom, eine (C₁ bis C₄)-Alkylgruppe, eine (C₁ bis C₄)-Monohydroxyalkylgruppe oder eine (C₂ bis C₄)-Polyhydroxyalkylgruppe und
- G²⁷ steht für ein Wasserstoffatom, eine gegebenenfalls substituierte Arylgruppe, eine (C₁ bis C₄)-Alkylgruppe oder eine (C₁ bis C₄)-Monohydroxyalkylgruppe, insbesondere für ein Wasserstoffatom oder eine Methylgruppe.

Bevorzugt bindet in Formel (E5) der Rest -NG²⁵G²⁶ an die 5 Position und der Rest G²⁷ an die 3 Position des Pyrazolzyklus.

Besonders bevorzugte Pyrazol-Derivate sind insbesondere die Verbindungen, die ausgewählt werden aus 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, sowie deren physiologisch verträglichen Salze.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazol, 2,4,5,6-Tetraamino-pyrimidin, 4-Hydroxy-2,5,6-triaminopyrimidin, 2-Hydroxy-4,5,6-triaminopyrimidin, sowie den physiologisch verträglichen Salzen dieser Verbindungen.

Im folgenden werden Beispiele für die als Substituenten der Verbindungen der Formeln (E1) bis (E6) genannten Reste aufgezählt: Beispiele für (C₁ bis C₄)-Alkylreste sind die Gruppen -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃. Erfindungsgemäße Beispiele für (C₁ bis C₄)-Alkoxyreste sind -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, -OC(CH₃)₃, insbesondere eine Methoxy- oder eine Ethoxygruppe.

Weiterhin können als bevorzugte Beispiele für eine (C₁ bis C₄)-Monohydroxyalkylgruppe -CH₂OH, -CH₂CH₂OH, -CH₂CH₂CH₂OH, -CHCH(OH)CH₃, -CH₂CH₂CH₂CH₂OH, wobei die Gruppe - CH₂CH₂OH bevorzugt ist.

Ein besonders bevorzugtes Beispiel einer (C₂ bis C₄)-Polyhydroxyalkylgruppe ist die 1,2-Dihydroxyethylgruppe.

Beispiele für Halogenatome sind F-, Cl- oder Br-Atome, Cl-Atome sind ganz besonders bevorzugte Beispiele.

Beispiele für stickstoffhaltige Gruppen sind insbesondere -NH₂, (C₁ bis C₄)-Monoalkylaminogruppen, (C₁ bis C₄)-Dialkylaminogruppen, (C₁ bis C₄)-Trialkylammoniumgruppen, (C₁ bis C₄)-Monohydroxyalkylaminogruppen, Imidazolinium und -NH₃⁺.

Beispiele für (C₁ bis C₄)-Monoalkylaminogruppen sind -NHCH₃, -NHCH₂CH₃, -NHCH₂CH₂CH₃, -NHCH(CH₃)₂.

Beispiele für (C₁ bis C₄)-Dialkylaminogruppe sind -N(CH₃)₂, -N(CH₂CH₃)₂.

Beispiele für (C₁ bis C₄)-Trialkylammoniumgruppen sind -N⁺(CH₃)₃, -N⁺(CH₃)₂(CH₂CH₃), -N⁺(CH₃)(CH₂CH₃)₂.

Beispiele für (C₁ bis C₄)-Hydroxyalkylaminoreste sind -NH-CH₂CH₂OH, -NH-CH₂CH₂OH, -NH-CH₂CH₂CH₂OH, -NH-CH₂CH₂CH₂OH.

Beispiele für (C₁ bis C₄)-Alkoxy-(C₁ bis C₄)-alkylgruppen sind die Gruppen -CH₂CH₂-O-CH₃, -CH₂CH₂CH₂-O-CH₃, -CH₂CH₂-O-CH₂CH₃, -CH₂CH₂CH₂-O-CH₂CH₃, -CH₂CH₂-O-CH(CH₃), -CH₂CH₂CH₂-O-CH(CH₃).

Beispiele für Hydroxy-(C₁ bis C₄)-alkoxyreste sind -O-CH₂OH, -O-CH₂CH₂OH, -O-CH₂CH₂CH₂OH, -O-CHCH(OH)CH₃, -O-CH₂CH₂CH₂CH₂OH.

Beispiele für (C₁ bis C₄)-Acetyaminoalkoxyreste sind -O-CH₂NHC(O)CH₃, -O-CH₂CH₂NHC(O)CH₃, -O-CH₂CH₂CH₂NC(O)CH₃, -O-CH₂CH(NHC(O)CH₃)CH₃, -O-CH₂CH₂CH₂CH₂NHC(O)CH₃.

Beispiele für (C₁ bis C₄)-Carbamoylaminoalkoxyreste sind -O-CH₂CH₂-NH-C(O)-NH₂, -O-CH₂CH₂CH₂-NH-C(O)-NH₂, -O-CH₂CH₂CH₂CH₂-NH-C(O)-NH₂.

Beispiele für (C₁ bis C₄)-Aminoalkylreste sind -CH₂NH₂, -CH₂CH₂NH₂, -CH₂CH₂CH₂NH₂, -CH₂CH(NH₂)CH₃, -CH₂CH₂CH₂CH₂NH₂.

Beispiele für (C₁ bis C₄)-Cyanoalkylreste sind -CH₂CN, -CH₂CH₂CN, -CH₂CH₂CH₂CN.

Beispiele für (C₁ bis C₄)-Hydroxyalkylamino-(C₁ bis C₄)-alkylreste sind -CH₂CH₂NH-CH₂CH₂OH, -CH₂CH₂CH₂NH-CH₂CH₂OH, -CH₂CH₂NH-CH₂CH₂CH₂OH, -CH₂CH₂CH₂NH-CH₂CH₂CH₂OH. Beispiele für Di[(C₁ bis C₄)-Hydroxyalkyl]amino-(C₁ bis C₄)-alkylreste sind -CH₂CH₂N(CH₂CH₂OH)₂, -CH₂CH₂CH₂N(CH₂CH₂OH)₂, -CH₂CH₂N(CH₂CH₂CH₂OH)₂, -CH₂CH₂CH₂N(CH₂CH₂CH₂OH)₂.

Ein Beispiel für Arylgruppen ist die Phenylgruppe.

Beispiele für Aryl-(C₁ bis C₄)-alkylgruppen sind die Benzylgruppe und die 2-Phenylethylgruppe.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Amino-benzomorpholin,
- Chinoxalinderivate wie beispielsweise 6-Methyl-1,2,3,4-tetrahydrochinoxalin,

Gemische aus zwei oder mehrer Verbindungen aus einer oder mehrerer dieser Klassen sind im Rahmen dieser Ausführungsform ebenso erfindungsgemäß.

Die erfindungsgemäß verwendbaren m-Aminophenole bzw. deren Derivate werden bevorzugt ausgewählt aus mindestens einer Verbindung der Formel (K1) und/oder aus mindestens einem physiologisch verträglichen Salz einer Verbindung gemäß Formel (K1), 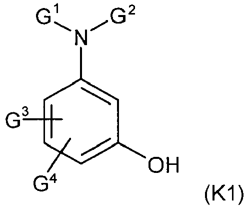 worin
- G¹ und G²: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₁ bis C₄)-Perfluoracylgruppe, eine Aryl-(C₁ bis C₄)-alkylgruppe, eine Amino-(C₁ bis C₄)-alkylgruppe, eine (C₁ bis C₄)-Dialkylamino-(C₁ bis C₄)-alkylgruppe oder eine (C₁ bis C₄)-Alkoxy-(C₂ bis C₆)-alkylgruppe, wobei G¹ und G² gemeinsam mit dem Stickstoffatom einen fünfgliedrigen, sechsgliedrigen oder siebengliedrigen Ring bilden können,
- G³ und G⁴: unabhängig voneinander stehen für ein Wasserstoffatom, ein Halogenatom, eine (C₁ bis C₄)-Alkylgruppe, eine (C₁ bis C₄)-Alkoxygruppe, eine Hydroxygruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe, eine (C₁ bis C₄)-Alkyox-(C₂ bis C₆)-alkoxygruppe, eine Arylgruppe oder eine Heteroarylgruppe.

Besonders bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Die erfindungsgemäß verwendbaren m-Diaminobenzole bzw. deren Derivate werden bevorzugt ausgewählt aus mindestens einer Verbindung der Formel (K2) und/oder aus mindestens einem physiologisch verträglichen Salz einer Verbindung gemäß Formel (K2), 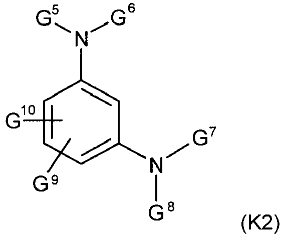 worin
- G⁵, G⁶, G⁷ und G⁸: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₁ bis C₄)-Alkoxy-(C₁ bis C₄)-alkylgruppe, eine Aryl-(C₁ bis C₄)-alkylgruppe, eine Heteroaryl-(C₁ bis C₄)-alkylgruppe, eine (C₂ bis C₄)-Perfluoracylgruppe, oder gemeinsam mit dem Stickstoffatom einen fünfgliedrigen oder sechsgliedrigen Heterozyklus bilden
- G⁹ und G¹⁰: unabhängig voneinander stehen für ein Wasserstoffatom, ein Halogenatom, eine (C₁ bis C₄)-Alkylgruppe, eine w-(2,4-Diaminophenyl)- (C₁ bis C₄)-alkylgruppe, eine w-(2,4-Diaminophenyloxy)- (C₁ bis C₄)-alkoxygruppe, eine (C₁ bis C₄)-Alkoxygruppe, eine Hydroxygruppe, eine (C₁ bis C₄)-Alkoxy-(C₂ bis C₄)-alkoxygruppe, eine Arylgruppe, eine Heteroarylgruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe.

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2'-hydroxyethyl)-aminobenzol und den physiologisch verträglichen Salzen aller vorstehend genannten Verbindungen.

Die erfindungsgemäß verwendbaren o-Diaminobenzole bzw. deren Derivate werden bevorzugt ausgewählt aus mindestens einer Verbindung der Formel (K3) und/oder aus mindestens einem physiologisch verträglichen Salz einer Verbindung gemäß Formel (K3), 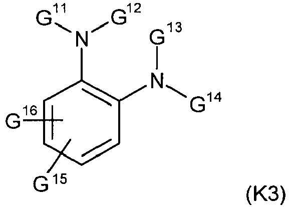 worin
- G¹¹, G¹², G¹³ und G¹⁴: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₁ bis C₄)-Alkoxy-(C₁ bis C₄)-alkylgruppe, eine Aryl-(C₁ bis C₄)-alkylgruppe, eine Heteroaryl-(C₁ bis C₄)-alkylgruppe, eine (C₂ bis C₄)-Perfluoracylgruppe, oder gemeinsam mit dem Stickstoffatom einen fünfgliedrigen oder sechsgliedrigen Heterozyklus bilden
- G¹⁵ und G¹⁶: unabhängig voneinander stehen für ein Wasserstoffatom, ein Halogenatom, eine Carboxylgruppe, eine (C₁ bis C₄)-Alkylgruppe, eine (C₁ bis C₄)-Alkoxygruppe, eine Hydroxygruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß verwendbaren Pyridinderivate werden bevorzugt ausgewählt aus mindestens einer Verbindung der Formel (K4) und/oder aus mindestens einem physiologisch verträglichen Salz einer Verbindung gemäß Formel (K4), 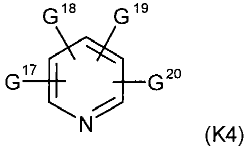 worin
- G¹⁷ und G¹⁸: stehen unabhängig voneinander für eine Hydroxygruppe oder eine Gruppe -NG²¹G²², worin G²¹ und G²² unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine Arylgruppe, eine (C₁ bis C₄)-Monohydroxyalkylgruppe, eine (C₂ bis C₄)-Polyhydroxyalkylgruppe, eine (C₁ bis C₄)-Alkoxy-(C₁ bis C₄)-alkylgruppe, eine Aryl-(C₁ bis C₄)-alkylgruppe, eine Heteroaryl-(C₁ bis C₄)-alkylgruppe,
- G¹⁹ und G²⁰: stehen unabhängig voneinander für ein Wasserstoffatom, ein Halogenatom, eine (C₁ bis C₄)-Alkylgruppe oder eine (C₁ bis C₄)-Alkoxygruppe.

Es ist bevorzugt, wenn gemäß Formel (K4) die Reste G¹⁷ und G¹⁸ in ortho-Position oder in meta-Position zueinander stehen.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß verwendbaren Indolderivate werden bevorzugt ausgewählt aus mindestens einer Verbindung der Formel (K5) und/oder aus mindestens einem physiologisch verträglichen Salz einer Verbindung gemäß Formel (K5), 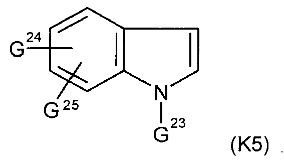 worin
- G²³: steht für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine Aryl-(C₁ bis C₄)-alkylgruppe,
- G²⁴: steht für eine Hydroxygruppe oder eine Gruppe -NG²⁶G²⁷, worin G²⁶ und G²⁷ unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe,
- G²⁵: Wasserstoffatom, ein Halogenatom oder eine (C₁ bis C₄)-Alkylgruppe,
mit der Maßgabe, dass G²⁴ in meta-Position oder ortho-Position zum Strukturfragment NG²³ der Formel bindet.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß verwendbaren Indolinderivate werden bevorzugt ausgewählt aus mindestens einer Verbindung der Formel (K6) und/oder aus mindestens einem physiologisch verträglichen Salz einer Verbindung gemäß Formel (K6), 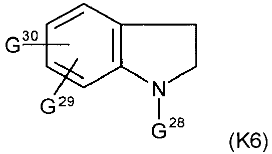 worin
- G²⁸: steht für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂bis C₄)-Polyhydroxyalkylgruppe, eine Aryl-(C₁ bis C₄)-alkylgruppe,
- G²⁹: steht für eine Hydroxygruppe oder eine Gruppe -NG³¹G³², worin G³¹ und G³² unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe,
- G³⁰: Wasserstoffatom, ein Halogenatom oder eine (C₁ bis C₄)-Alkylgruppe,
mit der Maßgabe, dass G²⁹ in meta-Position oder ortho-Position zum Strukturfragment NG²⁸ der Formel bindet.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Im folgenden werden Beispiele für die als Substituenten der Verbindungen der Formeln (K1) bis (K6) genannten Reste aufgezählt: Beispiele für (C₁ bis C₄)-Alkylreste sind die Gruppen -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃. Erfindungsgemäße Beispiele für (C₃ bis C₆)-Cycloalkylgruppen sind die Cyclopropyl, die Cyclopentyl und die Cyclohexylgruppe.

Erfindungsgemäße Beispiele für (C₁ bis C₄)-Alkoxyreste sind -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, -OC(CH₃)₃, insbesondere eine Methoxy- oder eine Ethoxygruppe.

Weiterhin können als bevorzugte Beispiele für eine (C₁ bis C₄)-Monohydroxyalkylgruppe -CH₂OH,-CH₂CH₂OH, -CH₂CH₂CH₂OH, -CH₂CH(OH)CH₃, -CH₂CH₂CH₂CH₂OH genannt werden, wobei die Gruppe -CH₂CH₂OH bevorzugt ist.

Ein besonders bevorzugtes Beispiel einer (C₂ bis C₄)-Polyhydroxyalkylgruppe ist die 1,2-Dihydroxyethylgruppe.

Beispiele für Halogenatome sind F-, Cl- oder Br-Atome, Cl-Atome sind ganz besonders bevorzugte Beispiele.

Beispiele für stickstoffhaltige Gruppen sind insbesondere -NH₂, (C₁ bis C₄)-Monoalkylaminogruppen, (C₁ bis C₄)-Dialkylaminogruppen, (C₁ bis C₄)-Trialkylammoniumgruppen, (C₁ bis C₄)-Monohydroxyalkylaminogruppen, Imidazolinium und -NH₃⁺.

Beispiele für (C₁ bis C₄)-Monoalkylaminogruppen sind -NHCH₃, -NHCH₂CH₃, -NHCH₂CH₂CH₃, -NHCH(CH₃)₂.

Beispiele für (C₁ bis C₄)-Dialkylaminogruppe sind -N(CH₃)₂, -N(CH₂CH₃)₂.

Beispiele für (C₁ bis C₄)-Alkoxy-(C₁ bis C₄)-alkylgruppen sind die Gruppen -CH₂CH₂-O-CH₃, -CH₂CH₂CH₂-O-CH₃, -CH₂CH₂-O-CH₂CH₃, -CH₂CH₂CH₂-O-CH₂CH₃, -CH₂CH₂-O-CH(CH₃)₂, -CH₂CH₂CH₂-O-CH(CH₃)₂.

Beispiele für (C₁ bis C₄)-Alkoxy-(C₁ bis C₄)-alkoxygruppen sind die Gruppen -O-CH₂CH₂-O-CH₃, -O-CH₂CH₂CH₂-O-CH₃, -O-CH₂CH₂-O-CH₂CH₃, -O-CH₂CH₂CH₂-O-CH₂CH₃, -O-CH₂CH₂-O-CH(CH₃)₂,
-O-CH₂CH₂CH₂-O-CH(CH₃)₂.

Beispiele für Hydroxy-(C₁ bis C₄)-alkoxyreste sind -O-CH₂OH, -O-CH₂CH₂OH, -O-CH₂CH₂CH₂OH, -O-CH₂CH(OH)CH₃, -O-CH₂CH₂CH₂CH₂OH.

Beispiele für (C₁ bis C₄)-Aminoalkylreste sind -CH₂NH₂, -CH₂CH₂NH₂, -CH₂CH₂CH₂NH₂, -CH₂CH(NH₂)CH₃, -CH₂CH₂CH₂CH₂NH₂.

Ein Beispiel für Arylgruppen ist die Phenylgruppe, die auch substituiert sein kann.

Beispiele für Aryl-(C₁ bis C₄)-alkylgruppen sind die Benzylgruppe und die 2-Phenylethylgruppe.

Die Gesamtmenge an Oxidationsfarbstoffvorprodukten in der wässrigen Zusammensetzung des Schritts (i) beträgt vorzugsweise 0,1 bis 20 Gew.-%, bezogen auf die gesamte wässrige Zusammensetzung.

Die wässrige Zusammensetzung enthält zusätzlich mindestens ein kationisches Polymer.

Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen. Homopolymere der allgemeinen Formel (P1), 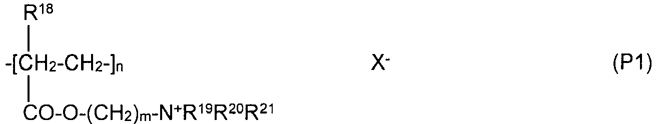 in der R¹⁸ = -H oder -CH₃ ist, R¹⁹, R²⁰ und R²¹ unabhängig voneinander ausgewählt sind aus C₁₋₄-Alkyl-, -Alkenyl- oder -Hydroxyalkylgruppen, m = 1, 2, 3 oder 4, n eine natürliche Zahl und X- ein physiologisch verträgliches organisches oder anorganisches Anion ist, sowie Copolymere, bestehend im wesentlichen aus den in Formel (P1) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁸ steht für eine Methylgruppe
- R¹⁹, R²⁰ und R²¹ stehen für Methylgruppen
- m hat den Wert 2.

Als physiologisch verträgliches Gegenionen X⁻ gemäß Formel (P1) kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid. Ein besonders geeignetes Homopolymer ist das, gewünschtenfalls vernetzte, Poly(methacryloyloxyethyltrimethylammoniumchlorid) mit der INCI-Bezeichnung Polyquaternium-37.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, wietere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (P1) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden.
- quaternierter Polyvinylalkohol,
sowie die unter den Bezeichnungen
- Polyquaternium 2 (z.B. Mirapol® A-15 der Firma Rhodia),
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von 5 · 105 bis 5 · 10⁶ (g/mol) auf.

Zur Herstellung erfindungsgemäßer Zubereitungen muß das Chitosan in die Salzform überführt werden. Dies kann durch Auflösen in verdünnten wäßrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Die kationischen Polymere sind in der wässrigen Zusammensetzung des Schritts (i) bevorzugt in einer Menge von 0,1 bis 5,0 Gew.-%, besonders bevorzugt von 0,25 bis 3,0 Gew.-%, jeweils bezogen auf die gesamte wässrige Zusammensetzung.

Die Gesamtmenge an Wasserstoffperoxid in der wässrigen Zusammensetzung des Schritts (i) beträgt vorzugsweise 1,5 bis 12 Gew.-%, insbesondere 2 bis 9 Gew.-%, jeweils bezogen auf die gesamte wässrige Zusammensetzung.

Eine trockene keratinhaltige Faser gemäß Schritt (ii) bzw. Schritt (iv) des erfindungsgemäßen Verfahrens liegt dann vor, wenn die den Haaren anhaftenden Wasserreste soweit entfernt wurden, dass das Haar zumindest nicht mehr tropfnass ist, das heißt eine fühlbare Restfeuchtigkeit im Haar verblieben ist. Haare mit diesem Trockenheitsgrad werden z.B. durch Handtuchtrocknung erhalten. Das Haar kann jedoch auch soweit getrocknet werden, dass der Feuchtigkeitsgehalt der Faser mit der Feuchtigkeit der Luft im wesentlichen im Gleichgewicht liegt oder die Faser Feuchtigkeit aus der Luft der Umgebung aufnimmt. Ein solche Trockenheitsgrad wird üblicherweise durch Trockung mit warmer Luft (z.B. mit einem Fön) erzielt.

Die in der wässrigen Zusammensetzung des Schritts (iv) enthaltenen keratinreduzierenden Verbindungen werden bevorzugt ausgewählt aus Verbindungen mit mindestens einer Thiolgruppe sowie deren Derivate, aus Sulfiten, Hydrogensulfiten und Disulfiten.

Verbindungen mit mindestens einer Thiolgruppe sowie deren Derivate sind beispielsweise Thioglykolsäure, Thiomilchsäure, Thioäpfelsäure, Phenylthioglykolsäure, Mercaptoethansulfonsäure sowie deren Salze und Ester (wie z.B. Isooctylthioglycolat und Isopropylthioglycolat), Cysteamin, Cystein, Bunte Salze und Salze der schwefligen Säure. Bevorzugt geeignet sind die Monoethanolammoniumsalze- oder Ammoniumsalze der Thioglykolsäure und/oder der Thiomilchsäure sowie die freien Säuren. Diese werden in der wässrigen Zusammensetzung bevorzugt in Konzentrationen von 0,5 bis 2,0 mol/kg bei einem pH-Wert von 5 bis 12, insbesondere von 7 bis 9,5, eingesetzt. Zur Einstellung dieses pH-Wertes enthalten die wässrigen Zusammensetzungen üblicherweise Alkalisierungsmittel wie Ammoniak, Alkali- und Ammonium-carbonate und - hydrogencarbonate oder organische Amine wie Monoethanolamin.

Beispiele für keratinreduzierende Verbindungen der Disulfite, die in der wässrigen Zusammensetzung enthalten sein können, sind Alkalidisulfite, wie z. B. Natriumdisulfit (Na₂S₂O₅) und Kaliumdisulfit (K₂S₂O₅), sowie Magnesiumdisulfit und Ammoniumdisulfit ((NH₄)₂S₂O₅). Ammoniumdisulfit kann dabei erfindungsgemäß bevorzugt sein. Beispiele für keratinreduzierende Verbindungen der Hydrogensulfite, die in der wässrigen Zusammensetzung enthalten sein können, sind Hydrogensulfite als Alkali-, Magnesium-, Ammonium- oder Alkanolammonium-Salz auf Basis eines C₂-C₄-Mono-, Di- oder Trialkanolamins. Ammoniumhydrogensulfit kann dabei ein besonders bevorzugtes Hydrogensulfit sein. Beispiele für keratinreduzierende Verbindungen der Sulfite, die in der wässrigen Zusammensetzung enthalten sein können, sind Sulfite als Alkali-, Ammonium- oder Alkanolammonium-Salz auf Basis eines C₂-C₄-Mono-, Di- oder Trialkanolamins. Ammoniumsulfit ist dabei bevorzugt. Der pH-Wert der wässrigen Zusammensetzung wird bei Verwendung von Sulfit und/oder Disulfit und/oder Hydrogensulft bevorzugt auf einen Wert im Neutralbereich von pH 5 bis 8, bevorzugt von pH 6 bis 7,5 eingestellt.

Bevorzugte C₂-C₄-Alkanolamine sind erfindungsgemäß 2-Aminoethanol (Monoethanolamin) und N,N,N-Tris(2-hydroxyethyl)amin (Triethanolamin). Monoethanolamin ist ein besonders bevorzugtes C₂-C₄-Alkanolamin, das insbesondere in einer Menge von 0,2 bis 6 Gew.-% bezogen auf die gesamte wässrige Zusammensetzung, eingesetzt wird.

Die in der wässrigen Zusammensetzung des Schritts (iv) enthaltenen keratinreduzierenden Verbindungen werden besonders bevorzugt ausgewählt aus Thioglykolsäure, Thiomilchsäure und Cystein sowie deren Salze.

Die keratinreduzierende Verbindung wird bevorzugt in einer Menge von 1 bis 25 Gew.-%, besonders bevorzugt in einer Menge von 4 bis 15 Gew.-%, bezogen auf die gesamte wässrige, keratinreduzierende Zusammensetzung, eingesetzt.

Darüberhinaus kann die wässrige, keratinreduzierende Zusammensetzung weitere Komponenten enthalten, die die Wirkung der keratinreduzierenden Verbindung auf das Keratin fördern. Solche Komponenten sind z.B. Quellmittel für keratinhaltige Fasern wie z.B. C₁-C₆-Alkohole und wasserlösliche Glykole oder Polyole wie z.B. Glycerin, 1,2-Propylenglykol oder Sorbit und Harnstoff oder Harnstoffderivate wie z.B. Allantoin und Guanidin sowie Imidazol und dessen Derivate. In einer bevorzugten Ausführung enthält die wässrige Zusammensetzung 0,05 bis 5 Gew.-% 1,2-Propylenglykol und/oder 0,05 bis 5 Gew.-% Harnstoff. Die Mengenangaben beziehen sich jeweils auf die gesamte wässrige Zusammensetzung.

In Schritt (vi) des erfindungsgemäßen Verfahrens werden die verformten keratinischen Fasern durch Aufbringen einer oxidierenden Zusammensetzung fixiert. Gleichzeitig gewährleistet das Aufbringen eines Oxidationsmittels, dass gegebenenfalls noch nicht abreagierte Oxidationsfarbstoffvorprodukte zu den entsprechenden Farbstoffen umgesetzt werden. Die in der oxidierenden Zusammensetzung enthaltene oxidierende Verbindung besitzt ein solches Redox-Potential, dass zwei Mercaptogruppen unter Bildung einer Disulfidbrücke oxidiert werden können. Geeignete Oxidationsmittel werden ausgewählt aus z.B. Natriumbromat, Kaliumbromat oder Wasserstoffperoxid. Es ist bevorzugt, Wasserstoffperoxid als Oxidationsmittel zu verwenden. Zur Stabilisierung wässriger Wasserstoffperoxidzubereitungen können zusätzlich übliche Stabilisatoren zugesetzt werden. Der pH-Wert der wässrigen H₂O₂-Zubereitungen, die anwendungsfertig üblicherweise etwa 0,5 bis 3,0 Gew.-% H₂O₂ enthalten, liegt bevorzugt bei 2 bis 6. Es kann auch von Konzentraten mit üblicherweise bis zu 30 Gew.-% H₂O₂ ausgegangen werden, die vor der Anwendung entsprechend verdünnt werden. Hierbei kann es bevorzugt sein, handelsübliche Schnellfixierungen, beispielsweise das Produkt Natural Styling Rinse Neutraliser 1:4 der Firma Henkel, einzusetzen. Enthält die oxidierende Zusammensetzung Bromat als Oxidationsmittel, so ist dies üblicherweise in Konzentrationen von 1 bis 10 Gew.-% enthalten und der pH-Wert der Lösungen wird auf 4 bis 7 eingestellt.

Sowohl die wässrige, keratinreduzierende Zusammensetzung, als auch die oxidierende Zusammensetzung können weiterhin alle für solche Zusammensetzungen üblichen, weiteren Inhaltsstoffe enthalten. Solche Inhaltsstoffe können beispielsweise Konditioniermittel, Tenside, Emulgatoren, UV-Stabilisatoren, Konservierungsmittel, Verdicker oder viskositätserhöhende Zusätze sein.

Vorzugsweise enthält die wässrige, keratinreduzierende Zusammensetzung und/oder die oxidierende Zusammensetzung mindestens einen oberflächenaktiven Stoff aus der Gruppe der anionischen, amphoteren, zwitterionischen und nichtionischen Tenside. Die Tenside haben unter anderem die Aufgabe, die Benetzung der Keratinoberfläche durch die Behandlungslösung zu fördern.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)_{X}-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 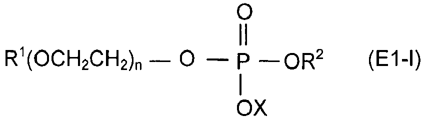 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C1 bis C4 - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Monoglyceridsulfate und Monoglyceridethersulfate der Formel (E1-III) 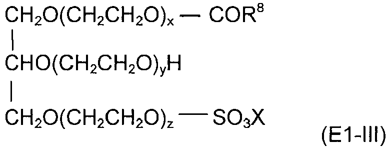 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht, wie sie beispielsweise in der EP 0 561 825 B1, der EP 0 561 999 B1, der DE 42 04 700 A1 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind,
- Amidethercarbonsäuren wie sie in der EP 0 690 044 beschrieben sind,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin können als nichtionische Tenside die Zuckertenside enthalten sein. Diese sind bevorzugt in Mengen von 0,1 bis 20 Gew.-%, bezogen auf die jeweilige gesamte Zusammensetzung, enthalten.

Mengen von 0,5 bis 15 Gew.-% sind besonders bevorzugt, und ganz besonders bevorzugt sind Mengen von 0,5 bis 7,5 Gew.%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tenside werden in Mengen von 0,1 bis 45 Gew.%, bevorzugt 0,5 bis 30 Gew.% und ganz besonders bevorzugt von 0,5 bis 25 Gew.%, bezogen auf die jeweilige gesamte erfindungsgemäß verwendete Zusammensetzung, eingesetzt.

In einer weiteren Ausführungsform können in den eingesetzten Zusammensetzungen Emulgatoren verwendet werden. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Weiterführende Definitionen und Eigenschaften von Emulgatoren finden sich in "H.-D. Dörfler, Grenzflächen- und Kolloidchemie, VCH Verlagsgesellschaft mbH. Weinheim, 1994". Erfindungsgemäß verwendbare Emulgatoren sind beispielsweise
- Anlagerungsprodukte von 4 bis 100 Mol Ethylenoxid und/oder 1 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z. B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die Emulgatoren werden bevorzugt in Mengen von 0,1 bis 25 Gew.-%, insbesondere 0,1 bis 3 Gew.-%, bezogen auf die jeweilige gesamte Zusammensetzung, eingesetzt.

Bevorzugt sind nichtionogene Emulgatoren mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen. Nichtionogene Emulgatoren mit einem HLB-Wert von 10 bis 16 können erfindungsgemäß besonders bevorzugt sein.

Die wässrige, keratinreduzierende Zusammensetzung und/oder die oxidierende Zusammensetzung können weiterhin mindestens einen linearen oder verzweigten, gesättigten oder ungesättigten Fettalkohol enthalten. Als Fettalkohole können eingesetzt werden Fettalkohole mit C₆-C₃₀-, bevorzugt C₁₀-C₂₂- und ganz besonders bevorzugt C₁₂-C₂₂- Gruppen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Die Fettalkohole werden beispielsweise in Mengen von 0,1 bis 20 Gew.-%, bezogen auf die gesamte Zubereitung, bevorzugt in Mengen von 0,1 bis 10 Gew.-% eingesetzt.

Die wässrige, keratinreduzierende Zusammensetzung und/oder die oxidierende Zusammensetzung können zusätzlich eine viskositätserhöhende Verbindung, im weiteren als Verdickungsmittel bezeichnet, enthalten.

Erfindungsgemäß einsetzbare Verdickungsmittel sind beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol, sowie viskositätserhöhende Polymere auf Polyacrylat-Basis wie sie beispielsweise unter den Handelsnamen Pemulen^{®}, Aculyn^{®} und Carbopol^{®} vertrieben werden. Desweiteren wird bevorzugt eine Mischung aus Diestern von 1,2-Propylenglykol mit Fettsäuren, beispielsweise der Verdicker mit der INCI-Bezeichnung Propylene Glycol Dicaprylate / Dicaprate, in den erfindungsgemäßen Mitteln eingesetzt.

Weiterhin können folgende Verbindungen enthalten sein:
- lineare und/oder verzweigte Fettsäuren, bevorzugt C₂-C₃₀-Fettsäuren, besonders bevorzugt C₄-C₁₈ Fettsäuren, am meisten bevorzugt C₆-C₁₀-Fettsäuren und/oder deren physiologisch verträglichen Salzen; weitere Beispiele sind Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Milchsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure,
- Polyhydroxyverbindungen; hierbei sind insbesondere zu nennen
   - Zucker mit 5 und/oder 6 Kohlenstoffatomen, insbesondere als Mono- und/oder Oligosaccharide, beispielsweise Glucose, Fructose, Galactose, Lactose, Arabinose, Ribose, Xylose, Lyxose, Allose, Altrose, Mannose, Gulose, Idose, Talose und Sucrose und/oder deren Derivate, z.B. Etherderivate, Aminoderivate und/oder Acetylderivate wie acetylierte Glucose, z.B. Tetraacetylglucose, Pentaacetylglucose und/oder 2-Acetamido-2-desoxyglucose. Bevorzugte Zuckerbausteine sind Glucose, Fructose, Galactose, Allose, Lactose, Arabinose und Sucrose; Glucose, Galactose und Lactose sind besonders bevorzugt;
   - Onsäuren, insbesondere Gluconsäure, Glucuronsäure;
   - Polyole, wie z.B. Glucamine, Glycerin, Mono- oder Diglyceride, 2-Ethyl-1,3-hexandiol, 2-Hydroxymethylpropantriol, Glycole wie Ethylenglykol, Diethylenglykol, Triethylenglykol, Propylenglykol, Dipropylenglykol, 1,3-Butandiol;
   - Polyhydroxysäuren, wie z.B. Pentahydroxyhexansäure, Tetrahydroxypentansäure und/oder deren Derivate, wie z.B. Ether, Ester und/oder Amide, z.B. Pentahydroxyhexansäureamid und/oder deren physiologisch verträglichen Salzen;_weitere Beispiele: Zitronensäure, Äpfelsäure oder Weinsäure;
- Pantolacton;
- Panthenol und/oder dessen Derivate;
- weitere Vitamine, wie z.B. Vitamin B6, C und/oder E und/oder deren Derivate;
- Hydroxysäuren, wie z.B. α-, β-Hydroxyfettsäuren bzw. Ketofettsäuren und/oder deren physiologisch verträglichen Salzen; wie beispielsweise Salicylsäure oder Milchsäure, Glyoxylsäure, Glycolsäure.
- wasserlösliche Polymere festigender Wirkung, z.B. Polyvinylpyrrolidon, Vinylacetat/Crotonsäure-Copolymere,
- Antischuppenwirkstoffe wie z.B. Picrotone Olamine, Zink Omadine,
- Wirkstoffe wie Allantoin, Pyrrolidoncarbonsäuren, Pflanzenextrakte ,
- pH-Einstell- und Puffermittel wie z.B. Citronensäure/Natriumcitrat, Ammoniumcarbonat, Ammoniumhydrogencarbonat, Guanidincarbonat, Ammoniak, Natriumhydroxyd,
- Komplexbildner wie EDTA, NTA, Organophosphonsäuren, Dipicolinsäure, Salicylsäure,
- Lichtschutzmittel (UV-Absorber),
- Öl-, Fett- und Wachskomponenten, bevorzugt in emulgierter Form,
- Farbstoffe, Trübungs- und Perlglanzmittel sowie
- gegebenenfalls Aerosol-Treibgase.

Die Erfindung wird anhand der folgenden Beispiele weiter erläutert, wobei die Beispiele das Verständnis des erfindungsgemäßen Prinzips erleichtern sollen und nicht als Einschränkung zu verstehen sind.

### Beispiele

Es wurden folgende Zusammensetzungen durch Vermischen der Inhaltsstoffe bereitgestellt:

### 1.1 oxidatives Färbemittel

Die im Folgenden beschriebene Färbecreme der Tabelle 2 wurde hergestellt und jeweils unmittelbar vor der Anwendung im Verhältnis 1:1 mit der folgenden Oxidationsmittelzubereitung vermischt:

**Tabelle 1: Oxidationsmittelzubereitung**

| | | | |
|---|---|---|---|
| 1. | Rohstoff | 2. | Gew.- |
| 3. | Dipicolinsäure | 4. | 0,1 |
| 5. | 50%ige KOH | 6. | 0,3 |
| 7. | Natriumbenzoat | 8. | 0,04 |
| 9. | Natriumpyrophosphat | 10. | 0,1 |
| 11. | Turpinal^{®} SL | 12. | 0,4 |
| 13. | 1,2-Propylenglykol | 14. | 0,4 |
| 15. | Cetyl/Stearylalkohol | 16. | 4,0 |
| 17. | Dehyquart^{®} B | 18. | 0,75 |
| 19. | Emulgin^{®} B2 | 20. | 1,2 |
| 21. | Paraffinöl | 22. | 0,3 |
| 23. | 50%ige H₂O₂ | 24. | 12,2 |
| 25. | Wasser | 26. | ad |

**Tabelle 2: Färbecreme**

| | | | |
|---|---|---|---|
| 27. | Rohstoff | 28. | F1 |
| 30. | Ammoniumcarbopollösung (1% in Wasser) | 31. | 15,0 |
| 32. | Lanette^{®} E | 33. | 0,7 |
| 34. | Laurylethersulfat (27% Aktivsubstanz) | 35. | 4,4 |
| 36. | Kaliumoleat (12.5% Aktivsubstanz) | 37. | 3,0 |
| 38. | Titandioxid | 39. | 0,5 |
| 40. | Cetylstearylalcohol 50/50 | 41. | 12,0 |
| 42. | Eumulgin^{®} B2 | 43. | 3,0 |
| 44. | Eutanol^{®} G | 45. | 2,0 |
| 46. | Cutina^{®} AGS | 47. | 2,0 |
| 48. | Cutina^{®} GMS-SE | 49. | 2,00 |
| 50. | Kalilauge 50% | 51. | 0,9 |
| 52. | EDTA, Tetranatriumsalz | 53. | 0,2 |
| 54. | Natriumsulfit | 55. | 0,2 |
| 56. | Ascorbinsäure | 57. | 0,05 |
| 58. | Merquat Plus^{®} 3330 | 59. | 1,5 |
| 60. | Parfum | 61. | 0,3 |
| 62. | Phospholipid^{®} EFA | 63. | 0,1 |
| 64. | Puricare^{®} LS 9658 | 65. | 0,1 |
| 66. | p-Aminophenol | 67. | 0,02 |
| 68. | p-Phenylendiamin | 69. | 1,15 |
| 70. | Resorcin | 71. | 0,60 |

**Fortsetzung Tabelle 2:**

| | | | |
|---|---|---|---|
| 72. | o-Aminophenol | 73. | 0,06 |
| 74. | m-Aminophenol | 75. | 0,15 |
| 76. | 2,6 Diaminopyridin | 77. | 0,01 |
| 78. | Ammoniaklösung (25% in Wasser) | 79. | ad pH 10,2 |
| 80. | Wasser | 81. | ad 100,0 |

- Ammoniumcarbopollsg.: Lösung eines Ammoniumsalzes eines Methacrylsäure-Methylacrylat-Copolymeren (INCI-Bezeichnung: Ammonium Polyacrylate) (Röhm GmbH)
- Cutina^{®} AGS: Ethylenglykoldistearat (INCI-Bezeichnung: Glycol Distearate) (Cognis)
- Cutina^{®} GMS-SE: INCI-Bezeichnung: Glyceryl Stearate SE (Cognis)
- Dehyquart^{®} B: Stearyltrimethylammoniumchlorid (ca. 60-66% Aktivsubstanzgehalt; INCI-Bezeichnung: Steartrimonium Chloride) (Cognis)
- Eumulgin^{®} B2: Cetylstearylalkohol mit ca. 20 EO-Einheiten (INCI-Bezeichnung: Ceteareth-20) (Cognis)
- Eutanol^{®} G: 2-Octyldodecylalkohol (INCI-Bezeichnung: Octyldodecanol) (Cognis)
- Lanette^{®} E: Fettalkoholsulfat-Natrium-Salz (ca. 90-96% Aktivsubstanz-gehalt; INCI-Bezeichnung: Sodium Cetearyl Sulfate) (Cognis)
- Merquat^{®} Plus 3330: Dimethyldiallylammoniumchlorid Acrylsäure Acrylamid Terpolymer (ca. 9,5% Festkörper in Wasser; INCI-Bezeichnung: Polyquaternium-39) (Ondeo-Nalco)
- Phopholipid^{®} EFA: (ca. 30% Festkörper in Wasser/Propylenglykol; INCI-Bezeichnung:Linoleamidopropyl PG-Dimonium Chloride Phosphate) (Uniqema)
- Puricare^{®} LS 9658: Moringa Pterygosperma Extrakt, in Wasser/Glycerin (ca. 1%ige Lösung; INCI-Bezeichnung: Water, Glycerine, Moringa Pterygosperma Seed Extract) (Cognis)
- Turpinal^{®} SL: 1-Hydroxyethan-1,1-diphosphonsäure (ca. 58 - 61% Aktivsubstanzgehalt; INCI-Bezeichnung: Etidronic Acid, Aqua (Water)) (Solutia)

### 1.2 Wellmittel

Das im Folgenden beschriebene Wellmittel der Tabelle 3 wurde hergestellt:

**Tabelle 3: Wellmittel**

| Rohstoff | Well |
|---|---|
| Eumulgin L ¹ | 0,80 |
| Plantapon ACG 35 ² | 0,80 |
| Natrosol^{®} 250 HR ³ | 1,25 |
| Ammoniak | 0,95 |
| Ammoniumthioglykolat (71%ige | 17,00 |
| Ammoniumbicarbonat | 8,00 |
| Polyquaternium-6 | 0,10 |
| Gluadin^{®} WQ ⁴ | 0,10 |
| Parfüm | 0,50 |
| Ammoniak (25 % wässrige Lösung) | 1,70 |
| Wasser | ad 100 |

| | |
|---|---|
| ¹ Laurylglykolether, ethoxyliert mit 1 Einheit Propylenoxid und 9 Einheiten Ethylenoxid (INCI-Bezeichnung: PPG-1-PEG-9 Lauryl Glycol Ether) (Cognis) ² N-Cocoyl-L-glutaminsäure Dinatriumsalz (31 Gew.-% Aktivsubstanz, INCI-Bezeichnung: Disodium Cocoyl Glutamate) (Cognis) ³ Hydroxyethylcellulose (Hercules) ⁴ Weizenproteinhydrolysat (ca. 31-35% Festkörper; INCI-Bezeichnung: Aqua (Water), Laurdimonium Hydroxypropyl Hydrolyzed Wheat Protein, Ethylparaben, Methylparaben) (Cognis) | |

### 1.3 Fixiermittel

Das im Folgenden beschriebene Fixiermittel der Tabelle 4 wurde hergestellt:

**Tabelle 4: Fixiermittel**

| Rohstoff | Fix |
|---|---|
| Aromox^{®} MCD-W ⁵ | 3,00 |
| Dehyquart^{®} A-CA ⁶ | 0,20 |
| Merquat^{®} 100 ⁷ | 0,25 |
| Methylparaben | 0,04 |
| Ortho-Phosphorsäure | 0,95 |
| Parfüm | 0,30 |
| Wasserstoffperoxid | 4,00 |
| Wasser | ad 100 |

| | |
|---|---|
| ⁵ N,N-Dimethyl-N-kokosalkylamin-N-oxid (ca. 30% Aktivsubstanz; INCI-Bezeichnung: Cocamine Oxide) (Akzo Nobel) ⁶ Trimethylhexadecylammoniumchlorid (ca. 24 -26 % Aktivsubstanz; INCI-Bezeichnung: Aqua (Water), Cetrimonium Chloride) (Cognis) ⁷ Poly(dimethyldiallylammoniumchlorid) (ca. 40% Festkörper; INCI-Bezeichnung: Polyquaternium-6) (Ondeo Nalco) | |

### 1.4 Zwischenspülung

Die im Folgenden beschriebene Zwischenspülung der Tabelle 5 wurde hergestellt:

**Tabelle 5: Zwischenspülung**

| Rohstoff | [Gew.-%] |
|---|---|
| Gluadin WQ | 1,00 |
| Natriumbenzoat | 0,10 |
| Glycin | 0,20 |
| Puricare LS 9658 | 0,10 |
| D-Panthenol | 0,50 |
| Luviquat FC 550 | 1,00 |
| Genamin CTAC | 0,50 |
| Milchsäure | 0,53 |
| Dow Corning 5225 C | 3,50 |
| Dow Corning 556 | 1,00 |
| Dow Corning 1501 | 3,50 |
| Parsol SLX | 1,00 |
| Parfum | 0,30 |
| Wasser | ad 100 |

- Genamin^{®} CTAC: Trimethylhexadecylammoniumchlorid (ca. 28-30% Aktivsubstanz in Wasser; INCI-Bezeichnung: Cetrimonium Chloride) (Clariant)
- Luviquat^{®} FC 550: 3-Methyl-1-vinylimidazoliumchlorid-Vinylpyrrolidon-Copolymerisat (50:50) (38-42% Festkörper in Wasser; INCI-Bezeichnung: Polyquaternium-16) (BASF)
- Dow Corning 1501: INCI-Bezeichnung: Cyclomethicone, Dimethiconol (ca. 85 % Cyclomethicone und ca. 15 % Dimethiconol) (Dow Corning)
- Dow Corning 556: Polyphenylmethylsiloxan (INCI-Bezeichnung: Phenyl Trimethicone) (Dow Corning)
- Dow Corning 5225 C: INCI-Bezeichnung: Cyclomethicone, PEG/PPG-18/18 Dimethicone (Dow Corning)
- Parsol^{®} SLX: Dimethicodiethylbenzal malonat (INCI-Bezeichnung: Polysilicone-15, CAS-Nr.: 207574-74-1) (DSM Nutritional Products)

### 2.0 Versuchsdurchführung des Halbseitenversuchs und Beurteilung der Ergebnisse:

Ungeschädigtes Haar wurde mit Wasser angefeuchtet und mit einem Handtuch frottiert.

Das anwendungsbereite oxidative Haarfärbemittel gemäß Punkt 1.1 wurde auf das Haar eines Probanden aufgetragen, dort über eine Einwirkzeit E1 von 20 Minuten belassen und danach ausgespült. Das Haar wurde mit dem Handtuch getrocknet.

Danach wurde auf der Hälfte des Probandenhaars eine Zwischenbehandlung mit der Zwischenspülung aus Punkt 1.4 durchgeführt und dort für 5 Minuten belassen und wieder ausgespült. Die andere Hälfte blieb unbehandelt.

Danach wurde das gesamte Haar auf Wickler gewickelt und mit dem Wellmittel aus Punkt 1.2 durchfeuchtet. Nach einer Einwirkzeit E2 von 20 Minuten wurde das Haar gespült und mit dem Handtuch getrocknet.

Danach wurde das Fixiermittel gemäß Punkt 1.3 auf dem gesamten Haar aufgetragen und über eine Einwirkzeit E3 von 10 Minuten im Haar belassen.

So dann wurden die Wickler entfernt, das Haar gespült und getrocknet.

Unabhängig von der Durchführung der Zwischenspülung wurde auf jeder Hälfte ein vergleichbares, sehr gleichmäßiges Well- und Färbeergebnis erzielt.

## Patentansprüche

1. Verfahren zum gleichzeitigen Färben und dauerhaften Verformen keratinhaltiger Fasern, insbesondere menschlicher Haare, wobei
die keratinhaltigen Fasern vor dem Schritt (i) angefeuchtet werden,
(i) eine wässrige Zusammensetzung, enthaltend mindestens ein Oxidationsfarbstoffvorprodukt, mindestens ein kationisches Polymer und Wasserstoffperoxid, auf die Fasern aufgetragen wird,
(ii) die Fasern nach einer Einwirkzeit Z1 gespült und gegebenenfalls getrocknet werden,
(iii) sodann die Fasern unter Zuhilfenahme von Verformungshilfsmitteln verformt werden,
(iv) sowie eine wässrige Zusammensetzung, enthaltend mindestens eine keratinreduzierende Verbindung (nachfolgend Wellmittel genannt) auf die Fasern aufgetragen wird,
(v) die Fasern nach einer Einwirkzeit Z2 gespült und gegebenenfalls getrocknet werden,
(vi) schließlich eine oxidierende Zusammensetzung, enthaltend mindestens eine oxidierende Verbindung (nachfolgend Fixiermittel genannt), auf die Fasern aufgetragen und nach einer Einwirkzeit Z3 wieder abgespült wird,
mit der Maßgabe, dass
- Schritt (iii) unmittelbar auf Schritt (ii) folgt,
- Schritt (iv) unmittelbar auf Schritt (iii) folgt oder die Schritte (iii) und (iv) gleichzeitig ablaufen.

2. Verfahren nach Anspruch 1, **dadurch gekennzeichnet, dass** die Oxidationsfarbstoffvorprodukte in einer Menge von 0,1 bis 20 Gew.-%, bezogen auf die gesamte wässrige, Zusammensetzung des Schritts (i), enthalten sind.

3. Verfahren nach einem der Ansprüche 1 bis 2, **dadurch gekennzeichnet, dass** die keratinreduzierende Verbindung ausgewählt wird aus mindestens einer Verbindung der Gruppe bestehend aus Thioglykolsäure, Thiomilchsäure, Thioäpfelsäure, Phenylthioglykolsäure, Mercaptoethansulfonsäure sowie deren Salzen und Estern, Cysteamin, Cystein, Bunte-Salzen und Salzen der schwefligen Säure, Alkalidisulfiten, wie z.B. Natriumdisulfit (Na₂S₂O₅) und Kaliumdisulfit (K₂S₂O₅), sowie Magnesiumdisulfit und Ammoniumdisulfit ((NH₄)₂S₂O₅), Hydrogensulfiten als Alkali-, Magnesium-, Ammonium- oder Alkanolammonium-Salze auf Basis eines C₂-C₄-Mono-, Di- oder Trialkanolamins, sowie Sulfiten als Alkali-, Ammonium- oder Alkanolammonium-Salze auf Basis eines C₂-C₄-Mono-, Di- oder Trialkanolamins.

4. Verfahren nach Anspruch 3, **dadurch gekennzeichnet, dass** die keratinreduzierende Verbindung ausgewählt ist aus Thioglykolsäure, Thiomilchsäure und Cystein sowie deren Salzen.

5. Verfahren nach einem der Ansprüche 1 bis 4, **dadurch gekennzeichnet, dass** die keratinreduzierenden Verbindungen in einer Menge von 1 bis 25 Gew.-%, bevorzugt 4 bis 15 Gew.-%, bezogen auf die gesamte wässrige, keratinreduzierende Zusammensetzung, enthalten sind.

6. Verfahren nach einem der Ansprüche 1 bis 5, **dadurch gekennzeichnet, dass** in der wässrigen, keratinreduzierenden Zusammensetzung zusätzlich mindestens ein anionisches, nichtionisches, amphoteres oder zwitterionisches Tensid enthalten ist.

7. Verfahren nach einem der Ansprüche 1 bis 6, **dadurch gekennzeichnet, dass** als oxidierende Verbindung Natriumbromat, Kaliumbromat oder Wasserstoffperoxid eingesetzt wird.

8. Verfahren nach einem der Ansprüche 1 bis 7, **dadurch gekennzeichnet, dass** als oxidierende Verbindung Wasserstoffperoxid eingesetzt wird.

9. Verfahren nach Anspruch 8, **dadurch gekennzeichnet, dass** Wasserstoffperoxid in einer Menge von 0,5 bis 3,0 Gew.-%, bezogen auf die gesamte oxidierende Zusammensetzung, enthalten ist.

## Claims

1. A method for simultaneously coloring and permanently shaping keratin-containing fibers, in particular human hair, wherein
the keratin-containing fibers are wetted prior to step (i),
(i) an aqueous composition, containing at least one oxidation dye precursor, at least one cationic polymer and hydrogen peroxide, is applied to the fibers,
(ii) the fibers are rinsed after a contact time Z1 and optionally dried,
(iii) the fibers are then shaped with the aid of shaping aids,
(iv) and an aqueous composition, containing at least one keratin-reducing compound (referred to as the waving agent below), is applied to the fibers,
(v) the fibers are rinsed after a contact time Z2 and optionally dried,
(vi) finally an oxidizing composition, containing at least one oxidizing compound (referred to as the fixing agent below), is applied to the fibers and rinsed off again after a contact time Z3,
provided that
- step (iii) takes place directly after step (ii),
- step (iv) takes place directly after step (iii), or steps (iii) and (iv) take place simultaneously.

2. The method according to claim 1, **characterized in that** the oxidation dye precursors are contained in an amount of from 0.1 to 20 wt.%, based on the total aqueous composition from step (i).

3. The method according to one of claims 1 to 2, **characterized in that** the keratin-reducing compound is selected from at least one compound from the group consisting of thioglycolic acid, thiolactic acid, thiomalic acid, phenyl thioglycolic acid, mercaptoethanesulfonic acid and salts and esters thereof, cysteamine, cysteine, Bunte salts and salts of sulfurous acid, alkali disulfites, such as sodium disulfite (NA₂S₂O₅) and potassium disulfite (K₂S₂O₅), and magnesium disulfite and ammonium disulfite ((NH₄)₂S₂O₅), hydrogen sulfites as alkali salts, magnesium salts, ammonium salts or alkanolammonium salts based on a C₂-C₄ mono-, di-or trialkanolamine, and sulfites as alkali salts, ammonium salts or alkanolammonium salts based on a C₂-C₄ mono-, di- or trialkanolamine.

4. The method according to claim 3, **characterized in that** the keratin-reducing compound is selected from thioglycolic acid, thiolactic acid and cysteine and salts thereof.

5. The method according to one of claims 1 to 4, **characterized in that** the keratin-reducing compounds are contained in an amount of from 1 to 25 wt.%, preferably 4 to 15 wt.%, based on the total aqueous, keratin-reducing composition.

6. The method according to one of claims 1 to 5, **characterized in that** at least one anionic, nonionic, amphoteric or zwitterionic surfactant is additionally contained in the aqueous, keratin-reducing composition.

7. The method according to one of claims 1 to 6, **characterized in that** sodium bromate, potassium bromate or hydrogen peroxide is used as the oxidizing compound.

8. The method according to one of claims 1 to 7, **characterized in that** hydrogen peroxide is used as the oxidizing compound.

9. The method according to claim 8, **characterized in that** hydrogen peroxide is contained in an amount of from 0.5 to 3.0 wt.%, based on the total oxidizing composition.

## Revendications

1. Procédé pour simultanément colorer et déformer des fibres de kératine, en particulier des cheveux humains, dans lequel :
on humidifie les fibres de kératine avant l'étape (i),
(i) on applique une composition aqueuse contenant au moins un précurseur de colorant d'oxydation, au moins un polymère cationique et un peroxyde d'hydrogène sur les fibres,
(ii) les fibres sont rincées après une durée d'action Z1, et éventuellement séchées,
(iii) ensuite, on déforme les fibres à l'aide d'adjuvants de déformation,
(iv) on applique également une composition aqueuse contenant au moins un composé réducteur de la kératine (désignée ci-dessous comme produit de mise en pli) sur les fibres,
(v) les fibres sont rincées après une durée d'action Z2, et éventuellement séchées,
(vi) enfin, une composition oxydante contenant au moins un composé oxydant (désignée ci-dessous comme fixateur) est appliquée sur les fibres et à nouveau rincée après une durée d'action Z3,
à condition que :
- l'étape (iii) se fasse immédiatement après l'étape (ii),
- l'étape (iv) se fasse immédiatement après l'étape (iii) ou les étapes (iii) et (iv) se fassent simultanément.

2. Procédé selon la revendication 1, **caractérisé en ce que** les précurseurs de colorant d'oxydation sont contenus à hauteur de 0,1 à 20 % en poids, rapporté à la composition aqueuse totale de l'étape (i).

3. Procédé selon une des revendications 1 à 2, **caractérisé en ce que** le composé réducteur de la kératine est choisi parmi au moins un composé du groupe constitué de l'acide thioglycolique, l'acide thiolactique, l'acide thiomalique, l'acide phénylthioglycolique, l'acide mercaptoéthanesulfonique, ainsi que leurs sels et esters, la cystéamine, la cystéine, les sels de Bunte et les sels de l'acide sulfureux, les bisulfites alcalins, ex. bisulfite de sodium (Na₂S₂O₅) et le bisulfite de potassium (K₂S₂O₅), ainsi que le bisulfite de magnésium et le bisulfite d'ammonium ((NH₄)₂S₂O₅), les hydrogénosulfites en tant que sels d'alcalins, de magnésium, d'ammonium ou d'alcanolammonium à partir d'une C₂₋₄-mono-, bi- ou trialcanolamine, ainsi que des sels d'alcalins, d'ammonium ou d'alcanolammonium à base de C₂₋₄-mono-, bi- ou trialcanolamine.

4. Procédé selon la revendication 3, **caractérisé en ce que** le composé réducteur de kératine est choisi parmi l'acide thioglycolique, l'acide thiolactique et la cystéine, ainsi que leurs sels.

5. Procédé selon une des revendications 1 à 4, **caractérisé en ce que** les composés réducteurs de kératine sont présents à hauteur de 1 à 25 % en poids, préférentiellement 4 à 15 % en poids, rapporté à la composition aqueuse réductrice de kératine totale.

6. Procédé selon une des revendications 1 à 5, **caractérisé en ce qu'**au moins un tensioactif anionique, non-ionique, amphotère ou zwitterionique est contenu dans la composition aqueuse réductrice de kératine.

7. Procédé selon une des revendications 1 à 6, **caractérisé en ce qu'**on utilise comme oxydant du bromate de sodium, du bromate de potassium ou du peroxyde d'hydrogène.

8. Procédé selon une des revendications 1 à 7, **caractérisé en ce qu'**on utilise comme oxydant du peroxyde d'hydrogène.

9. Procédé selon la revendication 8, **caractérisé en ce que** le peroxyde d'hydrogène est contenu à hauteur de 0,5 à 3,0 % en poids rapporté à la composition oxydante totale.
